# FASCICULE DE BREVET EUROPEEN

(11) **EP 0 671 912 B1**
(45) Date de publication et mention de la délivrance du brevet: **22.03.2000**
(21) Numéro de dépôt: 94900881.7
(22) Date de dépôt: 26.11.1993
(51) Int. Cl.: A61K 31/335, A61K 47/10, A61K 47/26

(54) **COMPOSITIONS INJECTABLES A BASE DE DERIVES DES TAXANES**
Injizierbare Arzneizubereitungen enthaltend Taxanderivate
INJECTABLE TAXANE DERIVATIVE BASED COMPOSITIONS

(30) Priorité: 02.12.1992 FR 9214501
(43) Date de publication de la demande: 20.09.1995
(73) Titulaire: RHONE-POULENC RORER S.A., 92160 Antony (FR)
(72) Inventeur: BOBEE, Jean-Marc, F-91370 Verrières-le-Buisson (FR); DE LANTY, Patrick, F-75009 Paris (FR); GUERIN, Gilles, F-95600 Eaubonne (FR); VEILLARD, Michel, F-92330 Sceaux (FR)
(74) Mandataire: Savina, Jacques
(86) Numéro de dépôt international: FR9301166
(87) Numéro de publication internationale: WO9412171

(56) Documents cités:
- EP-A- 0 118 316
- EP-A- 0 253 738
- EP-A- 0 522 937
- CHEMICAL ABSTRACTS, vol. 106, no. 22, 1 Juin 1987, Columbus, Ohio, US; abstract no. 182581c, & J.PARENTER.SCI.TECHNOL. vol. 41, no. 1 , 1987 pages 31 - 33 B.D.TARR ET AL. 'A new parenteral vehicle for the administration of some poorly soluble anti-cancer drugs'
- JOURNAL OF THE NATIONAL CANCER INSTITUTE vol. 82, no. 15 , 1 Aoüt 1990 pages 1247 - 1259 E.K.ROWINSKY ET AL. 'Taxol:a novel investigational antimicrotubule agent' cité dans la demande

## Description

La présente invention concerne une nouvelle forme pharmaceutique à base d'un agent thérapeutique ayant une activité antitumorale et antileucémique. Elle concerne plus particulièrement une nouvelle forme injectable contenant des taxoïdes tels que le taxol, le taxotère ou des dérivés de formule générale suivante :

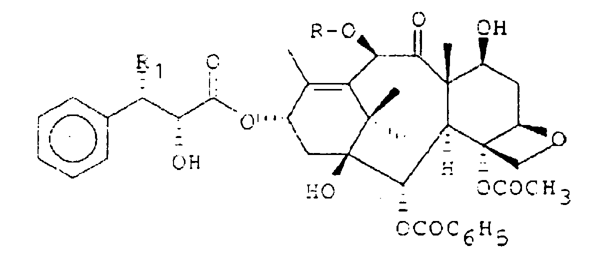

Dans la formule (I), R représente un atome d'hydrogène ou un radical acétyle, le symbole R₁ représente un radical tertiobutoxycarbonylamino ou benzoylamino. On préfère parmi l'ensemble de ces dérivés les deux dérivés pour lesquels R représente un groupe acétyle et R₁ un groupe benzoylamino ou celui pour lequel R représente un atome d'hydrogène et R₁ un radical tertio butoxycarbonylamino.

Le premier de ces deux composés est plus connu sous la dénomination de taxol, le deuxième est connu sous la dénomination de Taxotère.

Ces produits présentent in vivo une activité importante sur les tumeurs malignes ce qui a permis de les étudier dans le traitement des maladies résistantes à toutes les autres thérapies anticancéreuses.

Malheureusement ces produits présentent une solubilité dans l'eau tellement faible qu'il a été nécessaire de préparer une formulation pour preparation injectable à base d'agent tensioactif et d'éthanol. L'éthanol, est le meilleur solvant pharmaceutique qui permette de solubiliser les molécules répondant à la formule (I).

A titre d'exemple, selon la publication de Rowinsky, Lorraine, Cazenave et Donehower parue dans le Journal of the National Cancer Institute, vol. 82, No 15, pages 1247 à 1259, le 1er Août 1990, on prépare une première solution, dite "solution mère", contenant environ 6 mg/ml de taxol dans un mélange solvant composé de:
- 50 % en volume d'éthanol
- 50 % en volume de Crémophor EL®.

Lors de l'injection, cette solution est mélangée avec un liquide de perfusion contenant du chlorure de sodium ou du dextrose. Pour obtenir un mélange stable, d'un point de vue physique comme d'un point chimique, les auteurs de cet article disent qu'il faut limiter la concentration en principe actif dans le soluté de perfusion à des concentrations d'environ 0,03 à 0,6 mg/ml (voir publication précédente page 1251 colonne 1, troisième paragraphe).

Or il est souhaitable de pouvoir injecter des doses suffisantes de principe actif, pour cela les cliniciens désirent injecter des concentrations en principe actif comprises entre environ 0,3 et 1 mg/ml dans le liquide de perfusion, au delà de ces doses apparaissent des phénomènes de chocs anaphylactiques difficiles à maîtriser dus pour l'essentiel au Cremophor® (voir la publication de Rowinsky page 1250 deuxième colonne dernier paragraphe).

Toujours selon cette publication, pour obtenir de telles concentrations (entre 0,3 et 1mg/ml) il est nécessaire d'injecter des solutions contenant en même temps que le principe actif des concentrations en chacun des composés suivants, éthanol et surtout Crémophor®, d'environ 8 g pour 100 ml de solution de perfusion. Le traitement demandant souvent l'administration de doses élevées de principe actif et la concentration du principe actif dans la solution étant relativement faible l'injection de fort volume a pour effet de provoquer durant le traitement on plus des manifestations anaphylactiques des manifestations d'éthylisme.

Il a été découvert, selon la demande française déposée sous le numéro 91.08527, que la mise en oeuvre de formes pharmaceutiques tout à fait nouvelles permettaient soit de diminuer fortement les concentrations en éthanol, soit encore de supprimer totalement le Crémophor® et l'éthanol dans les solutés de perfusion.

Pour cela, selon un premier procédé de mise en oeuvre de cette demande de brevet, on préparait une solution mère contenant le principe actif dans un mélange de solvants composé d'éthanol qui est le meilleur solvant biocompatible des principes actifs de la classe des taxanes et d'un agent tensioactif choisi parmi les polysorbates commercialisés notamment sous les dénominations Tween® et Montanox®, ou les ester-éther d'oxyde d'éthylène et de glycérides d'acides gras (huile de ricin hydrogénée ou non) commercialisés par exemple sous la dénomination de Crémophor® ou d'Emulphor®.

La solution mère était préparée par dissolution du principe actif dans l'éthanol puis addition progressive de l'agent tensioactif. On pouvait ainsi préparer des solutions contenant 10 à 100 mg/ml de principe actif dans un mélange contenant environ 50 % d'agent tensioactif. L'éthanol contenu dans cette solution était ensuite éliminé au moins partiellement par évaporation sous vide ou par tout autre moyen approprié.

Selon un deuxième procédé de préparation de la solution mère, on dissolvait directement le principe actif dans l'agent tensioactif. Selon une meilleure manière de mettre en oeuvre l'invention, on préparait une solution de tensioactif contenant notamment 1 à 2 % d'éthanol et on ajoutait en continu le principe actif dans cette solution en agitant à l'aide par exemple d'un broyeur hélicoïdal ou d'une turbine dilacératrice. La présence d'une faible quantité d'éthanol apporte plusieurs avantages, le milieu présente une viscosité moins élevée, le mouillage de la poudre est amélioré ainsi que la filtration finale de la solution.

La solution mère, à faible teneur en éthanol, contient de préférence moins de 5 % d'éthanol, elle contient encore plus préférentiellement moins de 2 % d'éthanol. Cette solution est stable et peut ainsi contenir jusqu'à 200 mg/ml et de préférence jusqu'à 80 mg/ml de principe actif dans l'agent tensioactif.

La solution mère de taxol présentait selon cette invention une concentration comprise entre 6 et 20 mg/ml de principe actif dans l'agent tensioactif. La solution mère de Taxotère présentait de préférence une concentration comprise entre 20 et 80 mg/ml de principe actif dans l'agent tensioactif.

Ces solutions dans le tensioactif contenant éventuellement de faibles quantités d'éthanol pouvaient être dissoutes dans le soluté de perfusion mais moyennant une agitation extrêmement violente par exemple à l'aide d'un appareil type Vortex. Ce type d'appareillage n'existant pas dans tous les hopitaux, il était nécessaire de faciliter la mise en solution de la composition précédente et ceci est l'objet de la présente invention.

Une autre solution pour dissoudre la solution mère dans le soluté de perfusion consiste à chauffer aux alentours de 40°C l'ensemble. Mais dans ce cas, le composé de formule (I) est partiellement dégradé

La présente invention consiste donc à réaliser une composition intermédiaire à double compartiment, destinée à la préparation d'une solution pour perfusion, contenant :
- d'une part, une solution d'un dérivé de la classe des taxanes dans un agent tensioactif, et
- d'autre part, un additif de dilution permettant d'éviter la formation d'une phase gélifiée ou de casser la phase gélifiée formée lors du mélange de cette solution avec une solution aqueuse.
La composition exacte est définie dans les revendications.

Ces additifs sont choisis parmi l'ensemble des additifs qui sont capables de casser ou d'éviter la formation de la phase gélifiée qui est formée entre l'émulsifiant contenant le dérivé de la classe des taxanes et l'eau.

Parmi les additifs permettant de casser ou d'éviter la formation de cette phase gélifiée, on peut citer les dérivés ayant un poids moléculaire égal ou inférieur à environ 200. Parmi ces composés on préfère encore plus ceux qui sont porteurs d'au moins une fonction hydroxyle ou d'une fonction amine tels que les acides aminés.

On peut citer, à titre d'exemples de tels composés :
- l'éthanol
- le glucose
- le glycérol
- le propylène glycol
- la glycine
- le sorbitol
- le mannitol
- l'alcool benzylique
- les polyéthylène glycols.

On peut aussi utiliser des sels minéraux tels que le chlorure de sodium.

La quantité d'additif utilisée varie en fonction de la nature de l'additif, elle est de préférence supérieure à 6 % en poids par rapport à la masse d'agent tensioactif et encore plus préférentiellement supérieure à 15 % en poids pour les polyols tels que le glycérol, le glucose ou le sorbitol.

Les solutions des taxoïdes dans l'agent tensioactif avec la solution aqueuse de l'additif de dilution sont de préférence présentées dans des ampoules, des flacons ou un dispositif à double compartiment permettant le mélange extemporané des deux solutions au moment de l'injection dans la poche de perfusion.

Les perfusions de Taxotère ou de taxol sont ensuite injectées à l'homme à un débit prédéterminé en fonction de la quantité de principe actif que l'on veut injecter. On n'observe pas avec ces solutions les phénomènes de chocs anaphylactiques que l'on observait avec les solutions de l'art antérieur.

Ainsi ces dernières perfusions ont permis de diminuer, par rapport à l'art antérieur, les quantités d'agent tensioactif injectées à l'homme d'environ 80 %.

L'invention sera plus complètement décrite à l'aide des exemples suivants qui ne doivent pas être considérés comme limitatifs de l'invention.

### EXEMPLE 1

Préparation de la solution de taxoïdes selon la demande de brevet 91.08527

On dissout 32 g de Taxotère dans 340 ml d'éthanol absolu puis on ajoute 830 g de Polysorbate 80. On évapore l'éthanol au rotavapor à 30°C sous une pression de 15 mm de mercure (2000Pa) pendant 2 heures.

La solution obtenue est stable, elle contient 40 mg/ml de Taxotère.

1 ml de celle solution est mélangé avec 3 ml d'une solution aqueuse contenant en masse 70 % d'eau et 30 % de glycérol. Après agitation manuelle la dissolution est totale. Dans le cas ou le mélange eau/glycérol est remplacé par de l'eau seule on observe après agitation la formation d'un gel hétérogène. On obtient le même résultat, c'est-à-dire une solution fluide, en ajoutant seulement 2 ml de la solution aqueuse de glycérol.

### EXEMPLE 2

On répète l'exemple 1 en remplaçant la solution de glycérol par une solution aqueuse de glucose contenant 35 % en poids de glucose. Après agitation manuelle, la solution est fluide.

### EXEMPLES 3 à 4

On reproduit l'exemple 1 en remplaçant le Polysorbate par différents tensioactifs les résultats sont indiqués dans le tableau suivant :

| ESSAI | TENSIOACTIF | Mélange de dilution | Observation |
|---|---|---|---|
| 3 | Crémophor EL® | eau-glycérol (64/36) | fluide |
| 4 | Crémophor RH 40® | eau-glycérol (64/36) | fluide |
| C1 | Crémophor EL® | eau | prise en masse |
| C2 | Crémophor RH 40® | eau | prise en masse |

### EXEMPLES 5 A 12

On opère dans les mêmes conditions qu'à l'exemple 1 mais en mélangeant 1 g de Polysorbate 80 avec 1 g du mélange de dilution indiqué dans le tableau suivant ; on observe la nature de la phase liquide obtenue

| ESSAIS | MELANGE DE DILUTION (% en masse) | | PROPORTIONS additifs(s) -Tween 80 (% en masse) | | ASPECT DU MELANGE |
|---|---|---|---|---|---|
| 5 | eau | : 62 | glycérol | : 27,5 | fluide |
| | glycérol | : 38 | Tween 80® | : 72,5 | |
| 6 | eau | : 62 | sorbitol | : 27,5 | fluide |
| | sorbitol | : 38 | Tween 80® | : 72,5 | |
| 7 | eau | : 62 | PEG 200 | : 27,5 | fluide |
| | PEG 200 | : 38 | Tween 80® | : 72,5 | |
| 8 | eau | : 62 | glucose | : 27,5 | fluide |
| | glucose | : 38 | Tween 80® | : 72,5 | |
| 9 | eau | : 62 | propylène | | fluide |
| | propylène | | glycol | : 27,5 | |
| | glycol | : 38 | Tween 80® | : 72,5 | |
| 10 | eau | : 78 | NaCl | : 15,4 | fluide |
| | NaCl | : 22 | Tween 80® | : 84,6 | |
| 11 | eau | : 62 | glycérol | : 13,8 | fluide |
| | glycérol | : 19 | glucose | : 13,8 | |
| | glucose | : 19 | Tween 80® | : 72,4 | |
| 12 | eau | : 62 | glycérol | : 11,0 | fluide |
| | glycérol | : 15,2 | glucose | : 11,0 | |
| | glucose | : 15,2 | NaCl | : 5,5 | |
| | NaCl | : 7,6 | Tween 80® | : 72,5 | |

### EXEMPLES 13 A 14

A une solution de 6 g de Polysorbate 80, on ajoute x g d'additif et 4 ml d'eau, la fluidité du milieu est observée.

Les résultats sont indiqués dans le tableau suivant :

| essai | additif | résultat |
|---|---|---|
| 13 | alcool benzylique 0,5 g | fluide |
| 14 | glycine 0,4 g | fluide |
| 15 | glycol 1,90 g | fluide |
| 16 | éthanol 0,60 g | fluide |
| 17 | glycérol 0,53 g | fluide |
| | éthanol 0,53 g | |

## Revendications

1. Compositions injectables, à double compartiment, destinées à la préparation d'une solution pour perfusion contenant des dérivés de la classe des taxanes, composées
- d'une part, d'une solution à moins de 5 % d'éthanol contenant le dérivé de la classe des taxanes dans un agent tensioactif choisi parmi les polysorbates ou les esters-éthers d'oxyde d'éthylène et de glycérides d'acides gras, et
- d'autre part, d'un additif de dilution, choisi parmi les composés organiques présentant un poids moléculaire inférieur à 200 ou choisi parmi des sels minéraux, permettant d'éviter la formation d'une phase gélifiée ou de casser la phase gélifiée formée lors du mélange de cette solution avec une solution aqueuse,
ledit additif étant présent dans une proportion supérieure à 6 % en poids par rapport au poids d'agent tensio-actif,
lesdites compositions étant des compositions intermédiaires pour la préparation des solutions pour perfusion.

2. Compositions selon la revendication 1 caractérisées en ce que l'additif est choisi parmi les dérivés organiques porteurs d'un groupe hydroxyle ou d'une fonction amine.

3. Compositions selon la revendication 2 caractérisées en ce que l'additif est choisi parmi le glucose, le glycérol, le sorbitol, le mannitol, la glycine, le polyéthylène glycol, le propylène glycol, l'alcool benzylique, l'éthanol.

4. Compositions selon la revendication 1 caractérisées en ce que l'additif est le chlorure de sodium.

5. Compositions selon l'une quelconque des revendications précédentes caractérisées en ce que le dérivé de la classe des taxanes est choisi parmi les dérivés de formule (I) 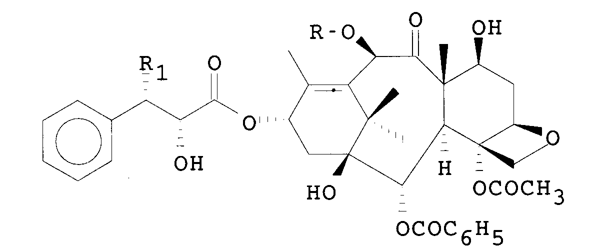 dans laquelle R représente un atome d'hydrogène ou un radical acétyle, le symbole R₁ représente un radical tertiobutoxycarbonylamino ou benzoylamino.

6. Compositions selon la revendication 5 caractérisées en ce que dans le composé de formule (I) R représente un groupe acétyle et R₁ représente un radical benzoylamino.

7. Compositions selon la revendication 5 caractérisées en ce que dans le composé de formule (I) R représente l'hydrogène et R₁ un radical tertiobutoxycarbonylamino.

8. Compositions selon l'une quelconque des revendications précédentes caractérisées en ce que le rapport pondéral entre l'additif et l'agent tensioactif est supérieur à 6 % et de préférence supérieur à 15 %.

9. Composition injectable selon l'une des revendications 1 à 8, caractérisée en ce que le mélange est préparé extemporanément au moment de l'emploi, à partir d'ampoules, de flacons ou d'un dispositif à double compartiment contenant d'une part la solution de dérivé de la classe des taxanes dans l'agent tensio-actif et d'autre part la solution aqueuse de l'additif de dilution.

## Claims

1. Injectable compositions, having a double compartment, intended for the preparation of a solution for infusion containing derivatives of the taxane class, which are composed
- on the one hand, of a solution with less than 5% ethanol containing the derivative of the taxane class in a surfactant chosen from polysorbates or the ether esters of ethylene oxide and of fatty acid glycerides, and
- on the other hand, of a dilution additive chosen from organic compounds having a molecular weight of less than 200 or chosen from inorganic salts and which makes it possible to avoid the formation of a gelled phase or to break the gelled phase formed during the mixing of this solution with an aqueous solution,
the said additive being present in a proportion greater than 6% by weight relative to the weight of surfactant,
the said compositions being intermediate compositions for the preparation of solutions for infusion.

2. Compositions according to claim 1, characterized in that the additive is chosen from organic derivatives carrying a hydroxyl group or an amine function.

3. Compositions according to claim 2, characterized in that the additive is chosen from glucose, glycerol, sorbitol, mannitol, glycine, polyethylene glycol, propylene glycol, benzyl alcohol and ethanol.

4. Compositions according to claim 1, characterized in that the additive is sodium chloride.

5. Compositions according to any one of the preceding claims, characterized in that the derivative of the taxane class is chosen from the derivatives of formula (I) 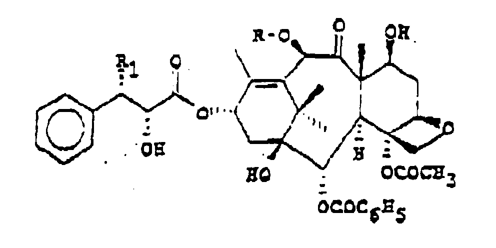 in which R represents a hydrogen atom or an acetyl radical, the symbol R₁ represents a tert-butoxy-carbonylamino or benzoylamino radical.

6. Compositions according to Claim 5, characterized in that in the compound of formula (I), R represents an acetyl group and R₁ represents a benzoylamano radical.

7. Compositions according to Claim 5, characterized in that in the compound of formula (I), R represents hydrogen and R₁ a tert-butoxycarbonylamino radical.

8. Compositions according to any one of the preceding claims, characterized in that the weight ratio between the additive and the surfactant is greater than 6% and preferably greater than 15%.

9. Injectable composition according to one of Claims 1 to 8, characterized in that the mixture is freshly prepared at the time of use from ampoules, vials or a double-compartment device containing, on the one hand, the solution of derivative of the taxane class in the surfactant and, on the other hand, the aqueous solution of the dilution additive.

## Patentansprüche

1. Injizierbare Zusammensetzungen mit zwei Kompartimenten zur Herstellung einer Infusionslösung, die Derivate der Klasse der Taxane enthält, bestehend aus
- einerseits einer Lösung mit mindestens 5 % Ethanol, die das Derivat der Klasse der Taxane in einem grenzflächenaktiven Mittel, ausgewählt aus Polysorbaten oder Ester-Ether-Ethylenoxiden und Fettsäureglyceriden, enthält, und
- andererseits einem Verdünnungsadditiv, ausgewählt aus organischen Verbindungen mit einem Molekulargewicht unter 200 oder ausgewählt aus mineralischen Salzen, die die Bildung einer gelartigen Phase verhindern oder die während des Mischens dieser Lösung mit einer wässerigen Lösung gebildete gelartigen Phase zerstören können,
wobei das Additiv in einem Anteil über 6 Gew.-% bezogen auf das Gewicht des grenzflächenaktiven Mittels vorhanden ist,
wobei die Zusammensetzungen intermediäre Zusammensetzungen für die Herstellung von Infusionslösungen sind.

2. Zusammensetzungen nach Anspruch 1, dadurch gekennzeichnet, daß das Additiv aus organischen Derivaten, die eine Hydroxylgruppe oder eine Aminfunktion tragen, ausgewählt ist.

3. Zusammensetzungen nach Anspruch 2, dadurch gekennzeichnet, daß das Additiv aus Glucose, Glycerin, Sorbit, Mannit, Glycin, Polyethylenglykol, Propylenglykol, Benzylalkohol, Ethanol ausgewählt ist.

4. Zusammensetzungen nach Anspruch 1, dadurch gekennzeichnet, daß das Additiv Natriumchlorid ist.

5. Zusammensetzungen nach einem der vorstehenden Ansprüche, dadurch gekennzeichnet, daß das Derivat der Klasse der Taxane aus den Derivaten der Formel(I) ausgewählt ist, 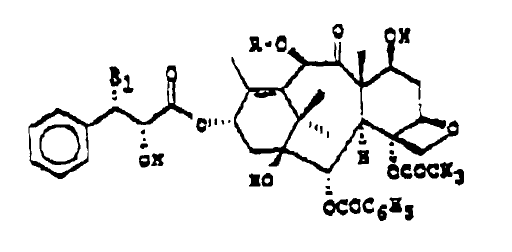 worin R ein Wasserstoffatom oder eine Acetylgruppe bezeichnet, das Zeichen R₁ einen tert.-Butoxycarbonylamino- oder Benzoylaminorest bezeichnet.

6. Zusammensetzungen nach Anspruch 5, dadurch gekennzeichnet, daß in der Verbindung der Formel (I) R eine Acetylgruppe bezeichnet und R₁ einen Benzoylaminorest bezeichnet.

7. Zusammensetzungen nach Anspruch 5, dadurch gekennzeichnet, daß in der Verbindung der Formel (I) R Wasserstoff bezeichnet und R₁ einen tert-Butoxycarbonylaminorest bezeichnet.

8. Verbindungen nach einem der vorstehenden Ansprüche, dadurch gekennzeichnet, daß das Gewichtsverhältnis zwischen dem Additiv und dem grenzflächenaktiven Mittel über 6 %, bevorragt über 15 % liegt.

9. Injizierbare Zusammensetzungen nach einem der Ansprüche 1 bis 8, dadurch gekennzeichnet, daß das Gemisch extamporär im Moment des Gebrauchs aus Ampullen, Fläschchen oder einer Vorrichtung mit zwei Kompartimenten, die einerseits die Lösung des Derivats der Klasse der Taxane in dem grenzflächenaktiven Mittel und andererseits die wässrige Lösung des Verdünnungsadditivs enthalten, hergestellt wird.
